# EUROPEAN PATENT APPLICATION

(11) **EP 3 447 490 A1**
(43) Date of publication of application: **27.02.2019**
(21) Application number: 17187943.0
(22) Date of filing: 25.08.2017
(51) Int. Cl.: G01N 33/49, A61B 5/157, G01N 11/06, A61B 5/02, G01N 11/00, G01N 13/00

(54) **ANALYSER FOR FLUID SAMPLE ANALYSIS**

(71) Applicant: Koninklijke Philips N.V., 5656 AE Eindhoven (NL)
(72) Inventor: VAN REENEN, Alexander, 5656 AE Eindhoven (NL)
(74) Representative: de Haan, Poul Erik

(57) **Abstract**

The present invention relates to an analyser for fluid sample analysis. It is described to receive (410) a fluid sample in a chamber. The fluid sample is moved (420) in the chamber over at least a predetermined distance. Physical changes are detected (430) in the chamber. A time for the fluid sample to move over the predetermined distance in the chamber is measured (440). At least one property of the fluid sample is determined (460) on the basis of the time for the fluid sample to move over the predetermined distance in the chamber.

## Description

### FIELD OF THE INVENTION

The present invention relates to an analyser for fluid sample analysis, system for fluid sample analysis, and to a method for fluid sample analysis, as well as to a computer program element and a computer readable medium.

### BACKGROUND OF THE INVENTION

Fluid sample analysis is performed using a disposable cartridge within which the fluid sample is supplied, and an analyser within which the cartridge is inserted for diagnostic testing. For a test, the cartridge is inserted into the analyser and a sample fluid is added to the sample interface of the cartridge. The sample fills a cartridge, after which the analyser starts the measurement. Quantitative results can be different for different sample types applied to such systems. Results can differ depending upon the sample properties (e.g. whole blood or blood plasma), the filling properties of the cartridge (in which the sample is introduced), and the stickiness of the analyte.

### SUMMARY OF THE INVENTION

It would be advantageous to have an improved technique for fluid sample analysis.

The object of the present invention is solved with the subject matter of the independent claims, wherein further embodiments are incorporated in the dependent claims. It should be noted that the following described aspects of the invention apply also for the analyser for fluid sample analysis, system for fluid sample analysis, and to the method for fluid sample analysis, as well as to a computer program element and a computer readable medium.

According to a first aspect, there is provided an analyser for fluid sample analysis, comprising:
- at least one detecting device arranged to detect physical changes; and
- a processing unit.

The analyser is arranged to be placed, positioned, arranged with respect to, or adapted to be in a close relation with or in an adjacent position with, a chamber or a container or a cavity configured to receive a fluid sample such that the at least one detecting device can detect physical changes in the chamber. This chamber, container or cavity may be typically provided as an arrangement distinct from the analyzer or be integrally part of the analyzer. The at least one detecting device is further arranged so as to measure a time for a fluid sample to move over a predetermined distance in the chamber. The processing unit is configured to determine at least one property of the fluid sample on the basis of the time for the fluid sample to move over the predetermined distance in the chamber.

In this way, a time for the fluid to flow over a distance, such as a length or width or depth of a chamber of a cartridge that interacts with the analyser, can be measured, and this can be used to determine one or more properties of a fluid sample.

In this way, the analyser can determine a property of a fluid sample without involvement of the user. For example, the analyser can differentiate one sample type from a different sample type, thereby enabling the correct analysis procedure for the determined sample type to be used. Also, the analyser can automatically correct for analysis results utilizing the time for the fluid sample to move a known distance, enabling for example the analyser to automatically account for different viscosities of a sample that can require adjustment of the results to obtain ground truth results.

In an example, the analyser comprises a memory storing at least one relation between fluid property and at least one measurement time.

In this way, the analyser can automatically determine the at least one fluid property on the basis of the time for the fluid flow to move over the predetermined distance.

In an example, the at least one detecting device comprises at least one radiation source and at least one detector.

In this was optical means can be used in measuring the time for the fluid sample to move over the predetermined distance.

In an example, the at least one radiation source and the at least one detector are configured to detect a presence of the fluid sample at an end position of the chamber to determine an end time relating to the time for the fluid sample to move over the predetermined distance in the chamber.

In other words, optical means can be used to determine a time when a chamber of for example a the cartridge has been filled up to a fixed (end) position, where the cartridge is transparent to radiation emitted by the at least one source to interact with fluid at the end position in the chamber of the cartridge, from which a time to fill the chamber up to that point can be determined.

In an example, the at least one radiation source and the at least one detector are configured to detect a presence of the fluid sample at a start position of the chamber to determine a start time relating to the time for the fluid sample to move over the predetermined distance in the chamber.

In other words, optical means can be used to determine a time when the chamber of for example a cartridge has begun to fill from which property of the fluid sample can be determined from a time for a fluid sample to flow over a predetermined distance of the chamber of the cartridge can be determined.

In an example, the analyser is further configured to store at least one threshold time, and wherein the processing unit is configured to use, directly or indirectly, this at least one threshold time to determine at least one property of the fluid sample on the basis of the measured time for the fluid sample to move over the predetermined distance in the chamber.

In other words, one or more properties of the fluid sample can be determined if the time for the fluid sample to move over the distance within the chamber of for example a cartridge is greater than a threshold time. And, different one or more properties of the fluid sample sample can be determined if the time for the fluid sample to move over the predetermined distance within the cartridge is less than a threshold time

In an example, the threshold time is determined and the processing unit is configured to determine that the fluid sample is a first type of fluid, among a plurality of types of fluids, when said measured time to move over the predetermined distance in the chamber is less than the threshold time.

In an example, the analyser comprises measurement tools to measure a first concentration of an analyte in the fluid sample. The analyser is further arranged to calibrate such measurement tools by taking into account said at least one property of the fluid sample.

According to a second aspect, there is provided a system for fluid sample analysis, comprising:
- an analyser for fluid sample analysis according to the first aspect and any of the associated examples; and
- a disposable having a chamber configured to receive a fluid sample.

According to a third aspect, there is provided a method for fluid sample analysis, comprising:
a) receiving a fluid sample in a chamber;
b) moving the fluid sample in the chamber over at least a predetermined distance;
c) detecting physical changes in the chamber;
d) measuring a time for the fluid sample to move over the predetermined distance in the chamber; and
f) determining at least one property of the fluid sample on the basis of the time for the fluid sample to move over the predetermined distance in the chamber.

In an example, step c) comprises transmitting radiation towards the chamber and detecting the radiation.

In an example, step c) comprises transmission of the radiation and the detection are arranged to detect a presence of the fluid sample at an end position of the chamber, and step d) comprises using the detected presence of the fluid sample at the end position of the chamber to determine an end time relating to the time for the fluid sample to move over the predetermined distance in the chamber.

In an example, step c) comprises radiation transmission and detection are arranged to detect a presence of the fluid sample at a start position of the chamber, and step d) comprises using the detected presence of the fluid sample at the start position of the chamber to determine a start time relating to the time for the fluid sample to move over the predetermined distance in the chamber.

In an example, step f) comprises using a threshold time to move over the predetermined distance in the chamber to determine at least one property of the fluid sample.

In an example, the method comprises step e) determining a first concentration of the fluid sample, and step f) comprises determining a second concentration of the fluid sample that is the first concentration that has been adjusted on the basis of the time for the fluid sample to move over the predetermined distance in the chamber.

According to another aspect, there is provided a computer program element for controlling a device or system as previously described which, when the computer program element is executed by a processing unit, is adapted to perform the method steps as previously described.

According to another aspect, there is provided a computer readable medium having stored the computer element as previously described.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of a device for use in fluid sample analysis;
Fig. 2 shows a schematic set up of an example of a device for use in fluid sample analysis;
Fig. 3a shows a schematic set up of an example of an analyser for fluid sample analysis;
Fig. 3b shows a schematic set up of an example of a system for fluid sample analysis;
Fig. 4 shows an example of a method for fluid sample analysis;
Fig. 5 shows a schematic set up of, and pictorial representation of, an example of a system for use in fluid sample analysis;
Fig. 6 shows a schematic set up of an example of a device for use in fluid sample analysis;
Fig. 7 shows a pictorial representation of parts of an example of a device for use in fluid sample analysis;
Fig. 8 shows a pictorial representation of a base part of an example of a device for use in fluid sample analysis;
Fig. 9 shows cumulative empirical probability distributions of observed fluid movement times over a distance of the device;
Fig. 10 shows an average plot of the data presented in Fig. 9;
Fig. 11 shows an example of a correction factor as the time for a fluid sample to move over a distance of the device;
Fig. 12 shows an example of blood-plasma correlation without correction; and
Fig. 13 shows the example of blood-plasma correlation shown in Fig. 12 that has been corrected on the basis of the time for a fluid sample to move over a distance of the device according to the correction shown in Fig. 11.

### DETAILED DESCRIPTION OF EMBODIMENTS

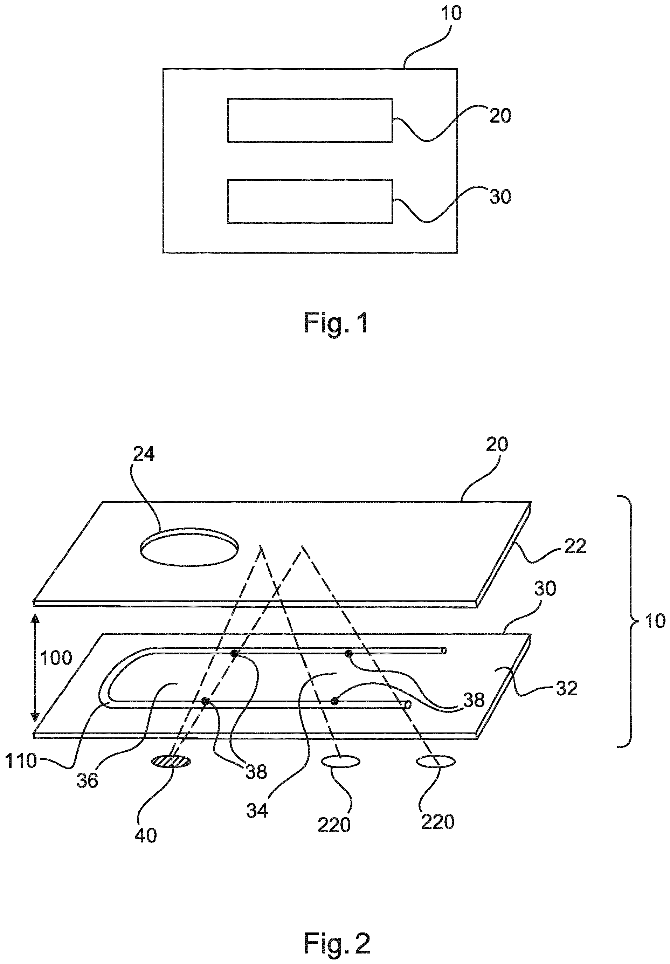

Figs 1 and 2 show examples of a device 10 for use in fluid sample analysis. The device can be termed a cartridge or disposable. The device 10 comprises a top part 20 and a base part 30. A bottom surface 22 of the top part is configured to be positioned adjacent to a top surface 32 of the base part. The top part 20 has an opening 24 through which a fluid sample can be supplied. When the top part 20 is positioned adjacent to the base part 30, a partially enclosed cavity is formed that connects with the opening 24 in the top part 20. The partially enclosed cavity has a length extending away from the opening 24. The device 10 is configured such that a fluid sample supplied through the opening 24 is moved laterally within the partially enclosed cavity over at least a part of the length of the partially enclosed cavity. The device 10 is configured such that a time for the fluid sample to move over the part of the length of the partially enclosed cavity can be measured. At least one property of the fluid sample is determinable on the basis of the time for the fluid sample to move over the part of the length of the partially enclosed cavity.

In this way, a time for the fluid to flow along a length can be measured, and this can be used to determine one or more properties of a fluid sample. The part of the length of the partially enclosed cavity over which the time for the fluid to move can be all of the length or a fraction of it, such as a half, quarter, three-quarters. Thus time itself to fill a fraction (that could be all) of the partially enclosed cavity, by moving along a length of the partially enclosed cavity, or a time derived parameter such as velocity of the fluid, can be used to determine at least one property of the fluid sample automatically without intervention of the user.

In other words, a partially enclosed cavity of the device has a characteristic length that extends away from the opening in a top part when the device is put together. A part of this length, that could be from the beginning of the partially enclosed cavity to the end of the partially enclosed cavity, or ¼ from the start of the partially enclosed cavity to the end of the partially enclosed or ½ from the start of the partially enclosed cavity to the end of the partially enclosed cavity or ¾ from the start of the partially enclosed cavity to the end of the partially enclosed cavity. A part of this length can also be from the start of the partially enclosed cavity to ¾, ½ or ¼ the end of the partially enclosed cavity or from ¼ from the start of the partially enclosed cavity to the end, ¾ or ½ way along the partially enclosed cavity. In other words, the part of the length of the partially enclosed cavity can include the beginning and end of the partially enclosed cavity, but it need not do. It just has to be a length of the partially enclosed cavity over which the time for the fluid sample can be measured. From this time measurement, which can in effect be a velocity measurement when the dimension of the part of the length is known, one or more properties of the fluid sample can be determined.

In this manner, the device is useable to differentiate between samples on the basis of a time to for the sample to move along at least a part of the partially enclosed cavity of the device. In this manner, a defined length to be filled that can be the entire cavity length can be determined and the time to fill this fixed length for one fluid sample can be objectively compared against the time to fill the same fixed length for a second fluid sample. Thus, for example, Blood can be differentiated from plasma, because blood has a higher viscosity than plasma and takes longer to flow along the length "and fill the device" than plasma. Also, plasma can be differentiated from water/watery buffers because plasma has a higher viscosity that water/watery buffers and takes longer to move along the length of the device. Also, saliva can be differentiated from water, because saliva has a higher viscosity than water and and takes longer to flow/move along the part of the length (fill a part of the device).

Additionally, frequently such devices contain wettable filters, similarly blood/plasma/serum/buffer/water can be differentiated from each other due to the different wetting characteristics of such fluids for example because the blood cells in blood fill the filter pores and slows the fluid flow, enabling blood to be differentiated from other fluids on the basis of a time for the fluid sample to move over a part of a length of the device. Also, when such a device uses a filter then saliva can be differentiated from buffer/water due to filter resistance differences for different fluids.

Fluid differentiation includes: Blood vs plasma vs buffer/water; based on viscosity (if no filter is present). Blood has a higher viscosity than plasma and plasma has a higher viscosity that water/watery buffers, and in this way these fluid samples can be differentiated one from the other on the basis of the time for those fluid samples to move along a part of a length of the device.

In this way, such devices can be used for the determination of concentration. Thus, the flow characteristics of the fluid can be taken into account when determining a concentration. In this way, a concentration determined for a fluid that has flowed slowly may be different to the correct concentration because operational characteristics of a reaction chamber used to determine concentration can change with the flow characteristics of the fluid. Then, on the basis of the time to flow along a fraction of a length of the device (fill or partially fill the device), a measure of the flow characteristics of the fluid can be determined and a measured concentration can be corrected to account for that flow characteristic. The same applies for fluid that has flowed quickly.

The top part and bottom part are termed as such in order for the skilled person to understand their relative positioning one to the other, and the skilled would understand that the bottom could have an opening through which the fluid sample can be supplied rather than the top. The fluid sample can even be supplied from the side if necessary, as again would be appreciated by the skilled person.

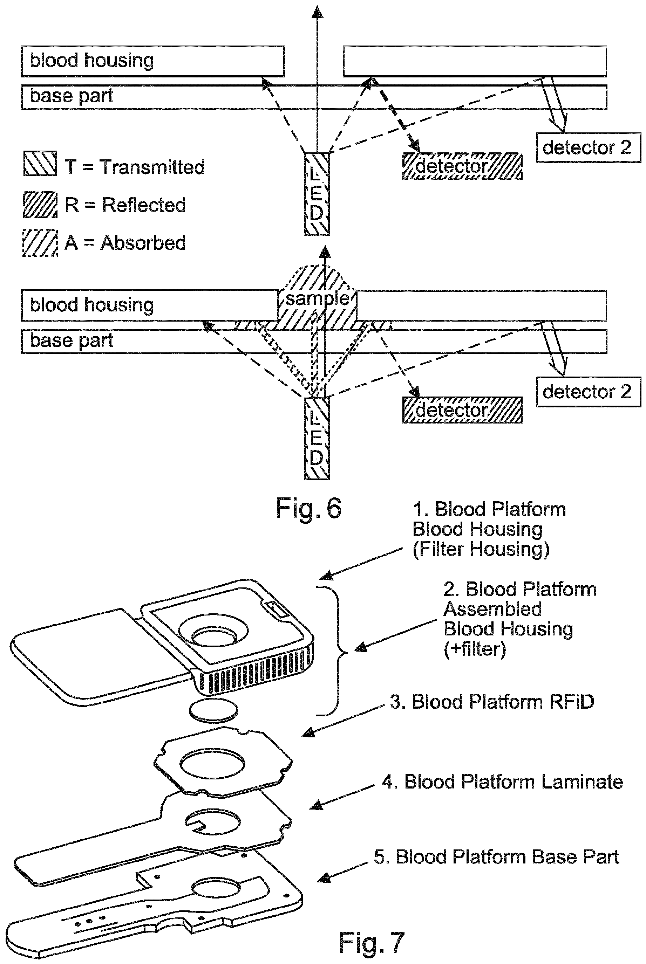

When the top part 20 is positioned adjacent to the base part 30 a fluid receiving region is defined there between, which is in the form of a partially enclosed cavity. The through opening 24 in the top part 20 enables fluid connection with the fluidic receiving region. A fluid sample is not shown in Fig 1 or 2, but can be seen in Fig. 6. The base part 30 comprises a fluid receiving surface 34, and a radiation window 36 extending over at least one or more parts of the fluid receiving surface 34. When the top part 20 is positioned adjacent to the base part 30, the device 10 is configured such that a fluidic sample supplied through the opening 24 is moved laterally in the fluid receiving region over the fluid receiving surface 34. This movement can be without the use of an intermediary membrane or can be with the use of an intermediary membrane. The membrane is the circular disc shown in Fig. 7. The radiation window 36 enables radiation from a source 40 to enter the device and be detected by a first detector 220. The source 40 and first detector and radiation window are configured to enable it to be determined when a fluid sample has is at a first position within the fluid receiving surface 34. The radiation window 36 also enables radiation from a source 40 to enter the device and be detected by a second detector 220. Here a source 40 is mentioned, which can be more than one source 40. The source 40 and second detector and radiation window are configured to enable it to be determined when a fluid sample has moved to a second position of the fluid receiving surface 34 of the partially enclosed cavity. The time for the fluid to move from the first position to the second position can then be determined, and this can be used to determine one or more properties of the fluid.

In an example, lateral movement of the fluid sample comprises spreading of the fluid sample. In an example, the fluid sample spreads in the fluid receiving region as the fluid sample is provided to the fluid receiving surface, such that the fluid sample extends substantially, or completely, back to the part of the fluid receiving surface to which is was first received.

In an example, lateral movement of the fluid sample comprises displacement of the fluid sample. In other words, the fluid sample can move as a droplet (which could be a flattened and/or elongated droplet) laterally in the fluid receiving region.

In an example, the through opening has an area of 5mm2. In an example, the through opening has an area of 1mm2. In an example, the through opening has an area of 2mm2. In an example, the through opening has an area of 25mm2. In an example, the through opening has an area less than 1mm2. The through opening can be circular, elliptical, rectangular, square, or any other shape as required.

In an example, when no sample is present a first radiation signal can be measured, and when a sample is present, and correctly presented within the device, a second (different) radiation signal can be measured and this enables the presence of the sample within the device to be determined. The second radiation signal could be a null signal indicating that all the radiation has been absorbed. In other words, a change in optical reflection properties of the device with and without a fluid sample is used to detect the sample. This detection technique can be used, with the two detectors, to determine that the sample is present at the first position and the second position.

In an example, a capillary channel runs within the top surface of the base part or the bottom surface of the top part, from a position within these respective surfaces close to the area of the opening, to an analysis zone. The capillary channel is configured to transfer some of the fluid sample to the analysis zone. Reference to movement of the sample within the fluid receiving region can mean movement within the capillary channel. Thus the capillary channel can form part of the partially enclosed cavity.

In an example, the fluid receiving region is configured such that lateral movement of a fluid sample is at least partly induced by a capillary force.

In an example, the spacing between the bottom surface of the top part and the top surface of the base part is dependent upon the specific fluid being analysed. For example, the spacing for blood can be different to the spacing for urine, with such spacing determined such that the flow of the fluid under capillary action is as required. In an example, the spacing between the bottom surface of the top part and the top surface of the base part is 0.05mm. In an example, the spacing between the bottom surface of the top part and the top surface of the base part is 0.1mm. In an example, the spacing between the bottom surface of the top part and the top surface of the base part can be 1mm, 2mm or any value between any of these values. In some situations, the spacing can be less than 0.05mm, and in other situations greater than 2mm.

In an example, the bottom surface of the top part is configured such that the flow of the sample across the bottom surface of the top part is not substantially inhibited.

In an example, the bottom surface of the top part is substantially flat.

In an example, bottom surface of the top part is hydrophobic.

In an example, the top surface of the base part is configured such that the flow of the fluid sample across the top surface of the base part is not substantially inhibited. In an example, the top surface of the base part is substantially flat. In an example, the top surface of the base part is hydrophobic.

In an example, a portion of the top surface of the base part is substantially flat.

In an example, a portion of the top surface of the base part is hydrophobic.

In this manner, the sample can spread out more effectively and quickly in less time and requiring a smaller volume. And if the bottom surface is positioned close enough to the top surface of the bottom part, additionally capillary action leads to more effective spreading of the fluid. This is further helped, if the bottom surface of the top part is also suitable flat.

In an example, the fluid receiving region is configured such that lateral movement of a fluidic sample is at least partly induced by a sucking action without the use of an active pump. In other words, the device is configured such that the fluid sample is sucked in the lateral direction (with respect to the central axis of the through hole), without an active pump.

In an example, fluidic guides are provided within or on the bottom surface of the top part to enable the sucking action of the fluid away from the central axis of the through hole. In an example, fluidic guides are provided within or on the top surface of the base part to enable the sucking action of the fluid away from the central axis of the through hole. In an example, the fluidic guides are formed as molded micro-grooves on or within the surfaces. In this way in addition to providing for increased movement of the fluid, which can augment or replace that caused by capillary action, the sucking action (through for example fluidic guides) can be used to better control the fluid flow. In this way, the increased flow can be directed toward the capillary channel that leads to the reaction chamber, and this means that less fluid needs to be applied to the device.

In an example, the bottom surface of the top part is hydrophilic. In an example, the top surface of the base part is hydrophilic. The surface of the top part and/or base part can be hydrophilized through application of a hydrophilic material or a material, such as for example Vitrostealth, which can be deposited on the surface. The spacing between the surface when one or both are hydrophilized can 0.05mm, 0.1mm, 1mm, 2mm or greater or less than these figures or between these figures. Regarding the situation when neither surface is hydrophilized, when one or both surfaces are hydrophilized, the spacing between surfaces may need to be changed for a particular fluid sample in order to provide for the required lateral movement of the fluid.

In this way, if the distance between the surfaces is small (< ∼1 mm), capillary forces can be the main mechanism leading to lateral movement of the sample (for example spreading). As the distance between the surfaces becomes larger, then the hydrophilicity of the top part or the base part can be used to also enable sufficient lateral movement of the sample (for example spreading), as the capillary mechanism reduces due to the increased separation. This means that flexibility of design is provided, where different distances between the surfaces of the device can be provided dependent upon the samples being analysed, with capillary and/or sucking action used to laterally move the fluid sample as required.

In an example, a sample container 110, or chamber, is provided on a bottom surface 22 of the top part 20 and/or on a top surface 32 of the base part 30, such that the fluid receiving region is a substantially parallel sided volume defined by the bottom surface of the top part, the top surface of the base part, and the sample container. The sample container 110 can also be called a partially enclosed cavity.

In an example, the sample container is formed within a vent ring. In an example, the vent ring forms an almost complete ring or annulus, with a gap for a capillary channel that runs to a sample analysis zone. The capillary channel is formed in the bottom surface of the top part or in the top surface of the base part.

In an example, the sample container is bounded by a horseshoe shape, being bounded by an annulus with an opening through which fluid can flow.

In an example, the sample container is bounded by a protrusion on the bottom surface of the top part, positioned around the opening in the top part.

In an example, the opening is oval shaped, with a long axis of the oval shape configured to be substantially parallel to an axis of the capillary channel. In this way, the oval shape confines the centre of delivery of the sample such that the centre of delivery does not occur to the side of the capillary channel. In an example, the chamber or sample container can be considered to be the capillary channel.

In an example, the fluid sample is blood. In an example, the fluid sample is serum. In an example, the fluid sample is urine. In an example, the fluid sample is water which may contain contaminants. In an example, the sample is a buffer.

In an example, when the top part 20 is positioned adjacent to the base part 30, a spacing 100 within the partially enclosed cavity between the top part and the bottom part is configured such that lateral movement of the fluid sample comprises a capillary movement.

In an example, the spacing is dependent upon the specific fluid being analysed. For example, the spacing for blood can be different to the spacing for urine, with such spacing determined such that the flow of the fluid under capillary action is as required.

In an example, the device comprises a filter. When the top part is positioned adjacent to the base part, at least a part of the filter is positioned within the partially enclosed cavity such that lateral movement of the fluid sample comprises a movement caused by wetting of the filter.

In an example, the at least one property comprises a concentration of the fluid sample.

In an example, the top part other than at the position of the opening and/or the base part comprises at least one radiation transparent portion, and wherein measurement of the time for the fluid sample to move over the part of the length of the partially enclosed cavity comprises utilisation of the at least one radiation transparent portion.

In this was optical means can be used to determine a fluid movement time of at least the part of the length of the partially enclosed cavity that makes use of a part of the device other than the hole through which the fluid sample is introduced. For example, radiation can be directed to the device in terms of visible radiation, such as a laser or LED, and this can be used to determine a time for fluid to move over a part of a length of the part of the partially enclosed cavity from which one or more properties of the fluid sample can be determined.

In an example, when the top part is positioned adjacent to the base part, the at least one radiation transparent portion comprises a portion that is located at least at a position at an end point of the part of the length of the partially enclosed cavity that is laterally spaced from the opening in the top part. The measurement of the time for the fluid sample to move over the part of the length of the partially enclosed cavity then comprises utilisation of the portion that is located at the position at the end point of the part of the length of the partially enclosed cavity to determine a time when the fluid sample has moved over the part of the length of the partially enclosed cavity.

In other words, optical means can be used to determine a time when the device has been filled up to a fixed point from which a time to fill the device up to that point can be determined.

In an example, the end point is at a known distance from the opening, such that a velocity of the fluid sample can be determined. This time derived parameter can also be used to determine at least one property of the fluid sample.

In an example, optical imaging is used to determine when the fluid sample has moved over the part of the length of the partially enclosed cavity. In an example, image-based analysis of optical images of a reaction chamber of the device is used to determine the time when the fluid sample has moved over a part of the length of the partially enclosed cavity, i.e. has been filled up to a known fixed point. The optical images can be obtained using frustrated total internal reflection of LED light at the bottom of the reaction chamber. The image based analysis can focus on specific features that become visible when the reaction chamber (which is at the end-point), such as the fluid front, or the optical broadening of the apparent chamber width due to the presence of fluid in the reaction chamber.

In an example, light can be reflected from the device and where the reflected light changes when fluid is present in comparison when it is not present, and this can be used to determine the time when the fluid has moved over a known length of the partially enclosed cavity.

According to an example, when the top part is positioned adjacent to the base part, the at least one radiation transparent portion comprises a portion that is located at least at a position at a start point of the part of the length of the partially enclosed cavity. The measurement of the time for the fluid sample to move over the part of the length of the partially enclosed cavity then comprises utilisation of the portion that is located at least at a position at the start point of the part of the length of the partially enclosed cavity to determine a time when the part of the length of the partially enclosed cavity has begun to be filled.

In an example, the at least one radiation transparent portion comprises a portion that is located at least at a position near to and/or opposite the opening in the top part.

In other words, optical means can be used to determine a time when the device has begun to fill from which property of the fluid sample can be determined from a time for a fluid sample to flow over a distance of the device (to fill a portion of the device) can be determined.

In an example, optical detection of a sample at the inlet of the cartridge is used to determine a time when the part of the length of the partially enclosed cavity has begun to fill. In an example, optical detection of a sample at a filter of the cartridge is used to determine a time when this part of the device has begun to fill

In an example, optical detection can done via reflection of light at the sample inlet. In an example, optical detection can comprises an image-based analysis.

In an example, the device comprises at least one fluid detecting electrode 38, and wherein measurement of the time for the fluid sample to move over the part of the length of the partially enclosed cavity comprises utilisation of the at least one fluid detecting electrode.

In other words, electrodes can be used to determine an end point in time of filling and/or a start point in time of filling from which a time to fill at least the part of length of the partially enclosed cavity of the device can be determined.

Thus, the device can use electrical detection for example to detect a start time when the fluid begins to move over the length of the partially enclosed cavity. Another electrode can be used to detect when the fluid sample has moved over the length, or an optical measurement can be used to detect this later time. However, an optical measurement can be used to detect the start time, and an electrode used to detect the later time. Additionally, electrodes and optical measurements can be used to detect both the start time and later times, if necessary, thereby providing redundancy of information and safeguarding against failure of a measurement technique.

In an example, one of the at least one electrode 38 is located at a position at an end point of the part of the length of the partially enclosed cavity that is laterally spaced from the opening in the top part. The measurement of the time for the fluid sample to move over the part of the length of the partially enclosed cavity comprises utilisation of the at least one electrode that is located at a position at an end point of the part of the length of the partially enclosed cavity to determine a time when the fluid sample has moved over the part of the length of the partially enclosed cavity. Fluid movement over the part of the length of the partially enclosed cavity can mean movement over or within a length of the capillary channel.

In other words, an electrode can be used to determine when the device has been filled from which a time to fill the device can be determined.

In an example, one of the at least one electrode 38 is located at a position at a start point of the part of the length of the partially enclosed cavity. The measurement of the time for the fluid sample to move over the part of the length of the partially enclosed cavity then comprises utilisation of the electrode that is located at the position at the start point of the part of the length of the partially enclosed cavity to determine a time when the part of the length of the partially enclosed cavity has begun to be filled.

In this manner, a time when the device starts to fill can be determined from which a time to fill at least the part of the device can be determined.

In an example, the at least one property of the fluid sample is determined on the basis of a threshold time to move over the part of the length of the partially enclosed cavity.

In an example, the at least one property of the fluid sample comprises a type of fluid. The fluid sample is determined to be a first type of fluid when the time to move over the part of the length of the partially enclosed cavity is less than the threshold time.

In an example, the fluid sample is determined to be a second type of fluid when the time to move over the part of the length of the partially enclosed cavity is greater than the threshold time.

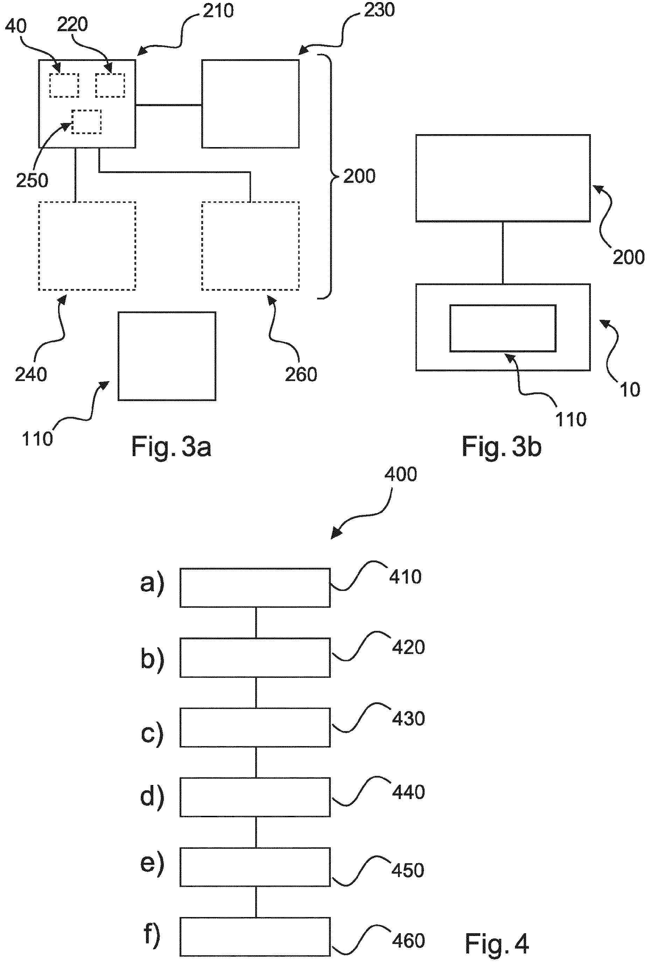

Fig. 3a shows an example of an analyser 200 for fluid sample analysis. The analyser 200 comprises at least one detecting device 210 arranged to detect physical changes, and a processing unit 230. The analyser 200 is arranged to be placed with respect to a chamber 110 configured to receive a fluid sample such that the at least one detecting device can detect physical changes in the chamber. The at least one detecting device 210 is further arranged so as to measure a time for a fluid sample to move over a predetermined distance in the chamber. The processing unit 230 is configured to determine at least one property of the fluid sample on the basis of the time for the fluid sample to move over the predetermined distance in the chamber.

In an example, the chamber 110 is a chamber of a cartridge, for example chamber 110 as shown in Fig. 2.

In an example, the chamber is a partially enclosed cavity of the cartridge.

In an example, the predetermined distance is a part of a length of the partially enclosed cavity as discussed with respect to Figs 1 and 2. In an example, the partially enclosed cavity connects with an opening within the cartridge through which the fluid sample can be supplied.

According to an example, the analyser comprises a memory 240 storing at least one relation between fluid property and at least one measurement time.

In an example, the at least one measurement time relates to times for a fluid or fluids to move over the predetermined distance of the chamber with the associated fluid properties of the fluid or fluids being stored in the memory. In another example, an algorithm is stored in the memory that is executed by the processing means, said algorithm being conceived so as to translate the measurement time into a fluid property based on said relation between fluid property and measurement time inherent to the algorithm..

According to an example, the at least one detecting device comprises at least one radiation source 40 and at least one detector 220.

According to an example, the at least one radiation source and the at least one detector are configured to detect a presence of the fluid sample at an end position of the chamber to determine an end time relating to the time for the fluid sample to move over the predetermined distance in the chamber.

In a specific example, there is no need of a start time measured by the detecting device, but a start time may be determined in different ways, such as for example it is determined when an event occurs with the fluid collection - e.g. a detected by the analyser via a mechanism or an interface arranged between the chamber or a micro fluidic path to the chamber (both embedded in a disposable cartridge) and the analyser, etc.

In an example, the end position is at a known distance from an opening in the chamber of a cartridge, such that a velocity of the fluid sample can be determined. This time derived parameter can also be used to determine at least one property of the fluid sample.

In an example, optical imaging is used to determine when the fluid sample has moved to the end point in the chamber. In an example, image-based analysis of optical images of a reaction chamber of the chamber of the cartridge is used to determine the time when the fluid sample has moved over the distance, such as the part of the length of the partially enclosed cavity, i.e. has been filled up to a known fixed point. The optical images can be obtained using frustrated total internal reflection of LED light at the bottom of the reaction chamber. The image based analysis can focus on specific features that become visible when the reaction chamber (which is at the end-point), such as the fluid front, or the optical broadening of the apparent chamber width due to the presence of fluid in the reaction chamber.

In an example, light can be reflected from the chamber of the cartridge and where the reflected light changes when fluid is present in comparison when it is not present, and this can be used to determine the time when the fluid has moved over a known length of the partially enclosed cavity.

In an example, detection of the presence of the fluid sample at the end position is based on a physical change in the chamber due to filling by the fluid sample. In an example, the physical change is a colour change.

According to an example, the at least one radiation source and the at least one detector are configured to detect a presence of the fluid sample at a start position of the chamber to determine a start time relating to the time for the fluid sample to move over the predetermined distance in the chamber.

In an example, optical detection of a sample at the inlet of a cartridge is used to determine a time when the chamber has begun to fill over the distance, for example the part of the length of the partially enclosed cavity has begun to fill. In an example, optical detection of a sample at a filter of the cartridge is used to determine a time when this part of the device has begun to fill

In an example, optical detection can be done via reflection of light at the sample inlet. In an example, optical detection can comprises an image-based analysis.

In an example, light can be reflected from the chamber of for example a cartridge and where the reflected light changes when fluid is present in comparison when it is not present, and this can be used to determine the time when the fluid has moved over the distance, such as over the a known length of the chamber such as over a length of a partially enclosed cavity.

In an example, detection of the presence of the fluid sample at the end position is based on a physical change in the chamber due to filling by the fluid sample. In an example, the physical change is a colour change.

In an example, the at least one detecting device comprises at least one sensor 250 configured to detect at least one signal from at least one fluid detection electrode 38 in the chamber of the cartridge.

In other words, sensors in the analyser can detect signals from electrodes in or associated with the chamber of the cartridge that can detect fluid, from which an end point in time of filling and/or a start point in time of filling of the distance of the chamber of the cartridge, such as a time to move over the predetermined distance, for example time to fillat least the part of length of the partially enclosed cavity of the cartridge, can be determined.

In an example, the at least one sensor is configured to detect a signal from a fluid detection electrode located at an end position of the chamber of the cartridge from which a presence of the fluid sample at the end position of the chamber of the cartridge can be determined to determine an end time relating to the time for the fluid sample to move over the distance in the chamber of the cartridge. The end position relates to the end of the predetermined distance of the chamber.

In other words, sensors can interact with an electrode in the cartridge to determine when the chamber of the cartridge has been filled from which a time to fill the cartridge can be determined.

In an example, the at least one sensor is configured to detect a signal from a fluid detection electrode located at a start position of the chamber of the cartridge from which a presence of the fluid sample at the start position of the chamber of the cartridge can be determined to determine a start time relating to the time for the fluid sample to move over the distance in the chamber of the cartridge. The start position relates to the start of the predetermined distance of the chamber.

In this manner, a time when the chamber of the cartridge starts to fill can be determined from which a time to fill at least the part of the chamber of the cartridge can be determined.

According to an example, the analyser is further configured to store at least one threshold time. The processing unit is configured then to use, directly or indirectly, this at least one threshold time to determine at least one property of the fluid sample on the basis of the measured time for the fluid sample to move over the predetermined distance in the chamber.

According to an example, the threshold time is determined and the processing unit is configured to determine that the fluid sample is a first type of fluid, among a plurality of types of fluids, when said measured time to move over the predetermined distance in the chamber is less than the threshold time.

In an example, the processing unit is configured to determine that the fluid sample is a second type of fluid, when the time to move over the predetermined distance in the chamber is greater than the threshold time.

According to an example, the analyser comprises measurement tools 260 to measure a first concentration of an analyte in the fluid sample. The analyser is further arranged to calibrate such measurement tools by taking into account said at least one property of the fluid sample.

In an example, adjustment of the first concentration comprises application of a first correction factor when the time to move over the predetermined distance, for example the part of the length of the partially enclosed cavity, is below a first time threshold.

In an example, adjustment of the first concentration comprises application of a second correction factor when the time to move over the predetermined distance, for example the part of the length of the partially enclosed cavity, is above the first time threshold.

In an example, adjustment of the first concentration comprises application of a third correction factor when the time to move over the predetermined distance, for example the part of the length of the partially enclosed cavity, is above a second time threshold.

In an example, the second correction factor is calculated on the basis of the first time threshold and the second time threshold. In an example, the second correction factor is calculated based on the first correction factor and the third correction factor. In an example, the second correction factor is equal to the first correction factor at the first time threshold and increases to equal the third correction factor at the second time threshold. In an example, the second correction factor linearly increases from the first time threshold to the second time threshold.

In an example, the means to determine the first concentration comprises a biochemical reaction.

Fig. 3b shows an example of a system 300 for fluid sample analysis. The system 300 comprises an analyser 200 for fluid sample analysis as described with respect to Fig. 3a, and a disposable 10 having a chamber 110 configured to receive a fluid sample. In an example, the disposable is the device also called a cartridge, as described with respect to Figs 1 and 2. In an example, the chamber is a sampled container 110 also called a partially enclosed cavity as described with respect to Figs 1 and 2.

Fig 4 shows a method 400 for fluid sample analysis in its basic step, where step e) is optional. The method 400 comprises:
- in a receiving step 410, also termed step a), receiving a fluid sample in a chamber;
- in a moving step 420, also termed step b), moving the fluid sample in the chamber over at least a predetermined distance;
- in a detecting step 430, also termed step c), detecting physical changes in the chamber;
- in a measuring step 440, also termed step d), measuring a time for the fluid sample to move over the predetermined distance in the chamber; and
- in a determining step 460, also termed step f), determining at least one property of the fluid sample on the basis of the time for the fluid sample to move over the predetermined distance in the chamber.

According to an example, step c) comprises transmitting radiation towards the chamber and detecting the radiation.

According to an example, in step c) transmission of the radiation and the detection are arranged to detect a presence of the fluid sample at an end position of the chamber. Step d) can then comprise using the detected presence of the fluid sample at the end position of the chamber to determine an end time relating to the time for the fluid sample to move over the predetermined distance in the chamber.

According to an example, in step c) radiation transmission and detection are arranged to detect a presence of the fluid sample at a start position of the chamber, and wherein step d) an comprise using the detected presence of the fluid sample at the start position of the chamber to determine a start time relating to the time for the fluid sample to move over the predetermined distance in the chamber.

In an example, step c) comprises using at least one sensor configured to detect at least one signal from at least one fluid detection electrode in the chamber of for example a cartridge.

In an example, step c) comprises using the at least one sensor to detect a signal from a fluid detection electrode located at an end position of the chamber from which a presence of liquid at the end position of the chamber can be determined. Step d) can then comprise using the detected presence of the fluid sample at the end position of the chamber to determine an end time relating to the time for the fluid sample to move over the predetermined distance in the chamber.

In an example, step c) comprises using the at least one sensor to detect a signal from a fluid detection electrode located at a start position of the chamber from which a presence of liquid at the start position of the chamber can be determined. Step d) can then comprise using the detected presence of the fluid sample at the start position of the chamber to determine a start time relating to the time for the fluid sample to move over the predetermined distance in the chamber.

According to an example, step f) comprises using a threshold time to move over the predetermined distance in the chamber to determine at least one property of the fluid sample.

In an example, step f) comprises determining that the fluid sample is a first type of fluid, of a plurality of possible types of fluids, when the time to move over the distance in the chamber of the cartridge is less than the threshold time.

In an example, step f) comprises determining that the fluid sample is a second type of fluid, when the time to move over the distance in the chamber of the cartridge is greater than the threshold time.

According to an example, the method comprises step e) determining 450 a first concentration of the fluid sample. Step f) can then comprise determining a second concentration of the fluid sample that is the first concentration that has been adjusted on the basis of the time for the fluid sample to move over the predetermined distance in the chamber.

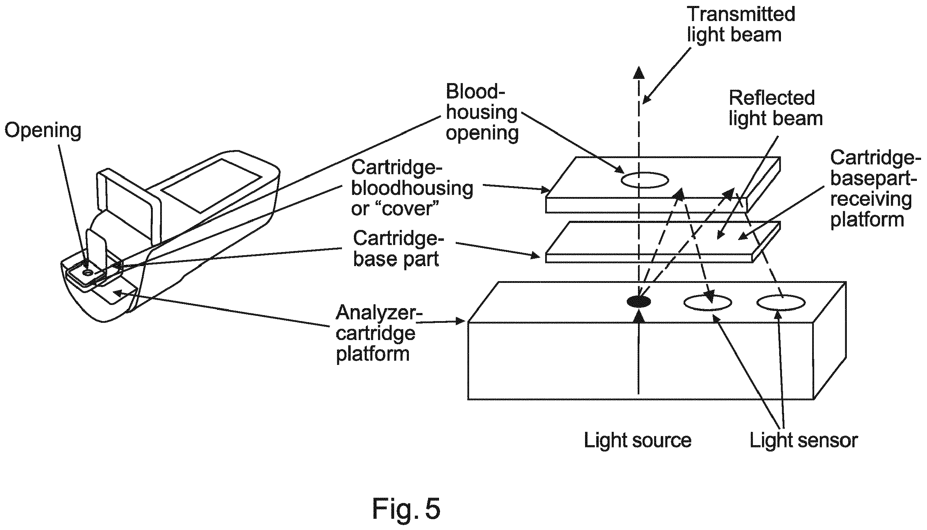

The analyser, system and method for fluid sample analysis are now described in more detail with reference to Figs 5-13.

Fig. 5 shows on the right hand side a schematic representation of a device, also termed a cartridge or disposable, for use in fluid sample analysis along with a representation of an analyzer, and on the left hand side a cartridge is shown inserted into an analyzer. In this example, the fluid sample being analyzed is blood. However, before analyzing the blood a check needs to be made that a sample is actually within the cartridge and is suitably positioned for analysis. A blood sample (not shown) is supplied to the cartridge through the blood housing opening in the cartridge blood housing (also called cover or top part) with a syringe or pipette. The blood sample is received on the cartridge base part receiving platform (base part). The top part is spaced from the base part such that the blood sample spreads laterally between the top part and the base part due to capillary action when no filter is present and when the filter is present the blood sample spreads laterally due to a combination of capillary action and wetting of the filter or can move due to wetting alone. The facing surfaces of the top part and base part, between which the blood flows, are smooth and hydroscopic in order to facilitate flow under capillary action. The blood flows to a capillary channel, which transfers some of the blood sample to a reaction chamber for analysis. To determine if the sample is present the device (cartridge) in inserted into the analyser. The analyser has a cartridge platform containing a light source (LED) and a light sensor. The LED is a white light LED, but can be a blue, red or green LED or can be a laser light source. A green light can be especially effective when the sample is blood, because green radiation is absorbed by blood. The base part of the device is partially transparent to the radiation emitted by the LED. The light from the LED passes through the base part, crosses the gap between the base part and the top part, is reflected from the underside of the top part, again crosses the gap between the top part and the base part, again passes through the base part and is detected by a light sensor (photodiode). The opening of the blood housing (top part) forms a sample inlet, and the sample spreads laterally due to capillary action as described above, with fluid moving across the top surface of the base part, which forms a fluid receiving surface. The above described light propagation route then means that light passes through the fluid receiving surface is reflected on a bottom surface of the top part at a radiation receiving area and again passes through the fluid receiving surface and in doing so passes twice through a region where the fluid sample can spread. Without a sample present, the detected intensity of radiation forms a baseline detected value. When a sample is supplied to the device, if the sample has flowed (or moved) due to capillary action over the region where radiation crosses between the base part and the top part, the detected intensity is reduced due to scattering and absorption losses. A comparison with the baseline intensity figure provides a reflectance value and this can be used to determine if the sample is present or not. In the above example, the sample is supplied through the opening in the top part of the device by means of a pipette or capillary tube. However, in an example, the upper surface of the top part has within it a microfluidic channel that leads to the opening, and which is used to transport the fluid sample to the opening and into the device. The fluid sample then enters the device and flows between the bottom surface of the top part and the top surface of the base part under capillary action as described above. Regarding sample detection, an example of the analyzer operates as follows: The LED provides radiation with pulses (on/off). The detector measures both of these pulses, and determines the difference. This signal difference changes upon adding sample. The on/off pulses are compared in order to prevent background light is causing a false trigger. For example, if someone would use a laser to shine upon the sensor, the detector will measure this intensity both during the on and off phase, and thereby not measure a difference. In the above description, the blood sample is thus determined to be in a part of the capillary channel, and a timer can start which then stops when the sample has laterally moved a set distance. Rather, than use a light based detection system, electrodes can be used to sense that the blood sample has started to flow within the capillary channel, as was described above with reference to Figs 1 and 2.

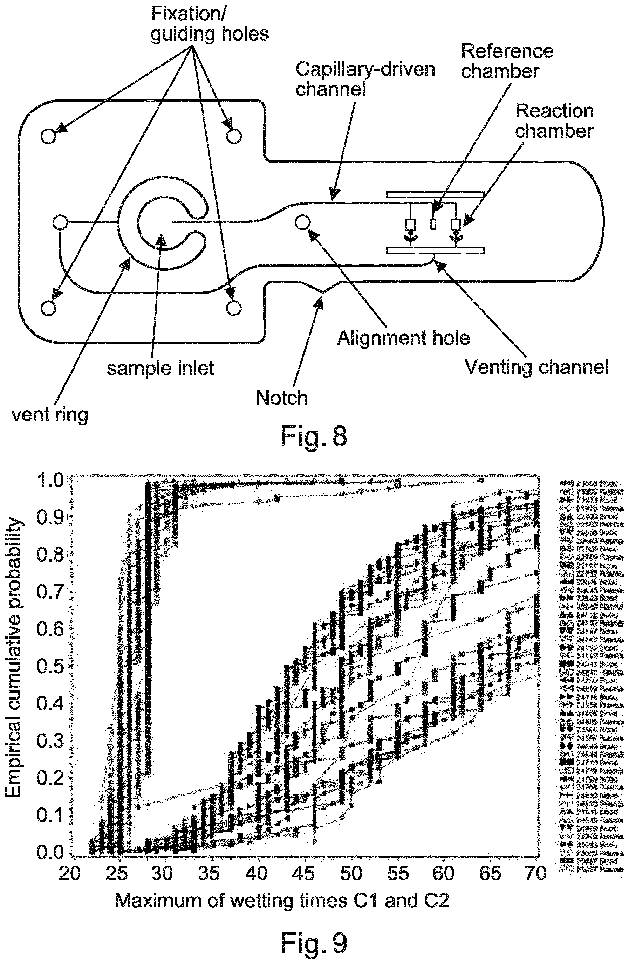

As shown schematically in Fig. 5, a second light sensor is positioned further away from the opening and is used to sense when the blood sample has moved a set distance, and at this point the timer is stopped, thereby providing a time for this particular blood sample to move from a start point of the capillary channel to a point further along the capillary channel. In Fig. 5 the second detector or light sensor is shown detecting radiation from the LED that illuminates the cartridge at this position further from the opening. A separate light source can be used, and if necessary no light source is required. The sample can be determined to have moved to this further position in a similar manner to that used to determine that the sample has started to flow into the capillary driven channel. However, in the specific embodiment shown, that the blood sample has moved the known distance to the reaction chamber is determined through image-based analysis of optical images of the reaction chamber, which is shown in Fig. 8. The optical images are obtained using frustrated total internal reflection of LED light at the bottom of the reaction chamber. The image based analysis focuses on specific features that become visible when the reaction chamber (which is the end-point of filling), such as the fluid front becoming visible, or the optical broadening of the apparent chamber width due to the presence of fluid in the reaction chamber. Upon detection that the blood sample is present the timer is stopped, providing a time stamp for the blood sample to have moved a set distance from the channel inlet to the reaction chamber. This time stamp, which in effect also indicates an average velocity of movement, can be used to determine a property of the blood sample, such as whether the sample is a blood sample or is actually plasma. This enables for example the sample to be determined to be blood, and a blood calibration used, or the sample to be determined to be plasma and a plasma calibration used. It is to be noted, that because blood cells are typically already filtered out at the sample application area, sample differentiation based on colour is not possible, for example via a colour based optical measurement at the reaction chamber. However, the fluid flow characteristics in terms of a flow velocity or time for the fluid to flow a distance can be used to differentiate between samples, and provide more information on that sample as discussed below.

As discussed in more detail with respect to Fig. 8, concentration measurements can be affected by the viscosity of the sample, which can skew (or bias) the concentration determined. However, by determining the movement time (in effect velocity of the sample) knowledge is provided about its viscosity and other flow related parameters, and this can be used to correct for the concentration result initially provided from the reaction chamber to provide a corrected concentration result, as well as differentiating one sample type from another.

Fig. 6 shows an expanded view of the device with and without a sample, wherein the figure shown no filter is present but a filter could be present. As shown, when no sample is present because the hole through which the sample is supplied is small the radiation from the source can reflect in an undisrupted manner within the device and be detected. However, even though the hole is small the sample flows under capillary action between the base part and blood housing (top part) and disrupts the reflectance due to absorption and scattering processes, and the fall in signal can be used to indicate that a sample is present and a timer can be started. A capillary channel extends off to one side of the base part (see Figs 7 and 8) and the radiation is reflected and detected on this side of the device. In the cartridge (device), the diameter of the sample opening of the blood-housing (top part) is small (reduced over that for existing devices) so that the contribution to the reflected light by the blood-housing (top part) is increased. Furthermore, the top part and base part are positioned relative to each other such that the fluid sample supplied through the small opening is laterally spread or displaced through capillary action between the base part and top part, or when a filter is present through a combination of capillary action and wetting of the filter. The fluid sample spreads over a region where radiation propagates to and from a light source to a first detector both located in a separate analyser that are positioned below the base part when the cartridge (device) is mounted in an analyser. The radiation propagates through the base part, reflects from the bottom of the top part, and propagates back through the base part to the detector. The surface over which the fluid sample spreads is large enough to disturb the reflected signal, providing a change in signal between a device with no sample present and that where a sample is present enabling a timer to start that indicates that the sample is present and has started to flow/move down the capillary channel. A second detector is positioned further downstream at the position of the reaction chamber, and as discussed above an image based analysis is used to determine when the sample is present at which time the timer is stopped. In Fig. 6 the fluid sample is shown only having started to flow down the capillary channel at which point the timer has just started, and when it has moved further down the channel and has been detected by the second detector the timer is stopped. Rather than use a detector to detect when the sample has moved to the reaction chamber, electrodes can be used.

Fig. 7 shows the component parts of a device for use in fluid sample analysis. The filter housing forms a top part, and in this figure has a large opening but the opening can be much smaller in size. In Fig. 7, the circular disc is a membrane or filter, which may or may not be present. The blood platform base part (base part) is also shown and a blood platform laminate and blood platform RFID are also shown. When the filter is not used, then the sample moves under capillary action and the speed of movement can be used to both determine the type of sample, and for a determined type of sample to then correct for the sample based on its flow velocity, for example correcting for viscosity effects on concentration measurements. When the filter is present, then again capillary action comes into play regarding movement of the fluid, and wetting of the filter also affects how the fluid moves. For example, for a filter plasma and blood move differently, with an influencing factor being the clogging of the filter with blood cells that can slow down the flow, as well as viscosity related effects. However, again on the basis of the determined flow time down the capillary channel to the reaction chamber, the sample types can be differentiated and then for a particular sample type the concentration corrected for viscosity induced effects, for example.

Fig. 8 shows the base part of a device for fluid analysis that can be used with or without a filtering membrane. The top part of the device (not shown) is positioned over the base part, such that the opening in the top part is centred over the horseshoe shaped vent ring. The capillary driven channel transfers sample that is supplied at the sample inlet to the analysis section comprising reaction and reference chambers. The two reaction chambers are filled with the sample fluid, and analysis of the sample fluid is undertaken. A pipette (or capillary tube) is used for transferring or supplying the sample through the opening in the top part of the device to the sample inlet. The capillary tube tip is directed towards the sample inlet, since the sample fluid must be in contact with the sample inlet to enable filling of the cartridge (device). The vent ring is provided for the venting of air, which prevents air bubble formation at the inlet, where such air bubbles can prevent cartridge filling. The vent ring also helps to confine the fluid within the fluid receiving surface defined within the vent ring. As discussed above, optical detection techniques are used to determine when the cartridge has started to fill, in particular when the capillary channel has started to fill and a timer starts, and when a reaction chamber has been filled, and the timer is stopped. Thus, the speed of flow/movement of the particular fluid sample can be determined as well as it being determined that the fluid sample both entered the device and then entered the reaction chamber. Also, as discussed above the sample type can be automatically determined as well as concentration corrections applied, using the speed of flow.

When the device (cartridge) is placed on the cartridge platform of the analyser (see Fig. 5) the cartridge platform is below the base part as shown in fig. 8. Radiation from the light source then passes through the base part within the area defined by the vent ring, which is transparent, or at least partially transparent, to the radiation emitted by the light source. This radiation propagates to and is reflected from a radiation receiving area on the underside of the top part of the device and again passes through the base part within the area defined by the vent ring. This radiation propagation route is directed towards the right-hand side of the area defined by the vent ring as shown in Fig. 8, towards the opening of the vent ring and the capillary driven channel. In this manner, it can be determined that the blood sample has started to flow into the capillary channel. Radiation from the light source also passes through the base part within the area at the positions of one or both of the reaction chambers, which is transparent, or at least partially transparent, to the radiation emitted by the light source. An imaging detector then images one or both reaction chambers, and determines if the blood sample is present due to changes in the images, such as detection of a flow front or refraction effects changing the apparent lateral dimension of the capillary channel.

The surface of the base part within the confines of the vent ring is flat and the material is hydrophobic. This means that the blood sample does not stick to the base part, but is able to flow across the base part between the base part and the top part under capillary action. This then defines what is meant by "flatness" of the surface, with this meaning that the surface does not have a roughness that inhibits to any great extent the flow or movement of the fluid. Similarly, the bottom surface of the top part of the cartridge (device) corresponding to the area over which the fluid sample flows is flat and material is hydrophobic. This aids the flow or movement of the sample, and the radiation receiving surface is then located within this flat region on the underside of the top part and this enhances or increases the reflectivity of the surface. In examples, the material need not be classed as hydrophobic, but can still permit flow or movement of the fluid sample. The reflectivity of the surface is further increased over the radiation receiving surface through that area been coloured white. This is a very cost-effective way of increasing reflectivity, however other means of increasing reflectivity could be used such as a mirrored area or an area having a reflectivity specifically tuned to the wavelength(s) of the light source. When the filter is used, the movement of the fluid can be via wetting of the filter, and it is not then necessary that the cartridge surfaces be flat and/or hydrophobic.

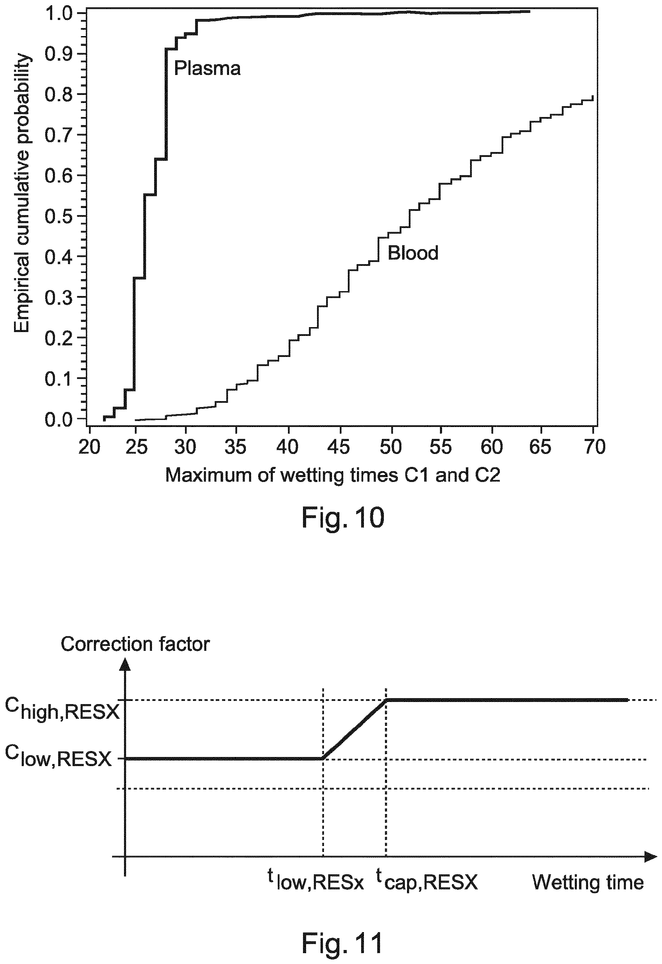

Fig. 9 shows the cumulative probability distribution of observed total wetting time for a data-set of 24 cartridge lots and in total 5056 measurements (plasma + blood combined). Fig. 10 shows the data of Fig. 9 as averaged data. Thus, for example a filling time of 30 seconds means that the sample has approximately a 98% probability of being plasma and a 2% probability of being blood. This enables a threshold time to be set, such as for example 30 seconds for blood vs plasma. Below a flow time of 30 seconds the sample can be determined, at high probability to be plasma, and above a flow time of 30 seconds the sample can be determined, again with a high probability, to be blood. Examples of sample differentiation are:
- Blood vs plasma/serum/buffer/water; based on filter resistance differences (blood cells fill the filter pores and slow fluid flow). Buffer type of fluids can be applicable for so-called quality control fluids that are used to test quality of a test system.
- Blood vs plasma vs buffer/water; based on viscosity (if no filter is present). Blood has a higher viscosity than plasma and plasma has a higher viscosity that water/watery buffers.
- Saliva vs buffer/water; if a filter is used, which can be different to that used for blood/plasma analysis, filter resistance differences can again be used for sample differentiation. If no filter is used, sample differentiation based on fluid velocity (flow time) can still be made based on viscosity, because saliva has a higher viscosity than buffer/water.

### Concentration measurement for a particular sample type

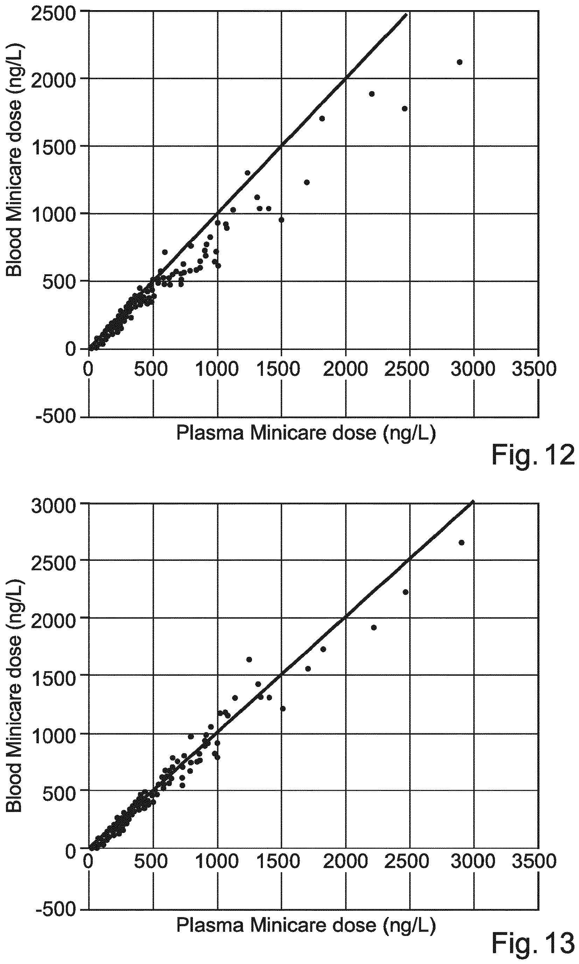

Even when the fluid flow characteristics have been used to determine the sample type, for example to determine if the sample is blood or plasma, the fluid flow characteristics can affect a determined concentration value. However, the determined flow time (speed) can be used to correct this determined concentration value, as now discussed. The concentration of analytes, in for example a Philips Minicare, is determined by performing a biochemical reaction in which specific molecules are used to specifically bind to the analyte to be detected. These specific molecules can be antibodies. These antibodies are attached to the cartridge, for example the bottom of the reaction chamber. Another set of antibodies that bind to another part of the analyte are used to attach a label to the analyte. This other set of antibodies are, in the Minicare, attached to magnetic beads (500 nm diameter). In a biochemical reaction, the analyte can bind first to the first antibodies on the bottom of the reaction chamber, and in a subsequent step magnetic beads bind to the captured analytes. Alternatively, the beads first capture an analyte, and in a second step bind the analyte to the reaction chamber surface. In this way a complex is formed. After the biochemical reaction, magnetic fields are used to move unbound magnetic particles away from the reaction chamber surface. Then the analyzer measures the amount of magnetic beads at the surface via frustrated total internal reflection of light that is directed from outside the cartridge to the reaction chamber bottom surface. Without magnetic beads at the bottom surface, the incident light is reflected from the surface. With magnetic beads at the bottom surface, the incident light is frustrated at the surface (or: interface), and less light is reflected from the surface, causing a change in signal. So, in this way, a higher magnetic bead concentration at the surface (due to a higher analyte concentration) causes a larger signal change, and thereby provides sensitivity of the measurement to the analyte concentration.
However, quantitative results can be different for different sample types applied to the system. This can be due to a different behaviour of the test system due to differences in filling speeds of the cartridge. For example, in the Minicare system, for a slow filling cartridge, the filling behaviour is different than for a fast filling cartridge. For example, the magnetic beads might disperse more for a slow filling sample, and lead to less binding in the assay and thereby a lower response. Thus components in the test system (here: magnetic beads) that are used in the concentration measurement (see previous point) are affected by the filling behavior of the fluid (by the fluid properties itself). For the specific case of the magnetic beads, the result of the concentration measurement above depends on the relative concentration of beads above the detection area (typically a spot of antibodies on the bottom of the reaction chamber). If this concentration changes, it can cause a bias in the measurement result. The bead concentration can change due to different filling behavior of the sample. This can be understood as follows: In a dried cartridge, magnetic beads are dried at the ceiling of the reaction chamber (a dried dot of beads). When the chamber fills with fluid, the beads will slowly disperse in the fluid. The location where the beads end up depends then on the speed of filling. A fast filling sample can lead to the transport of few beads from one location to another, because the fluid has stopped flowing before the beads are dispersed. A slow filling sample can transport more beads to another location, because beads are dispersing while the fluid flow has not yet stopped. As a result, the concentration of beads on the detection area can be different depending upon fluid flow speeds, leading to a measurement bias. This effect has been described for magnetic beads but also can relate to any reagent in the cartridge that dissolves in the sample. Furthermore, changes in properties/behavior of components need not be induced by the filling time, but can also be induced by the different properties of different sample types (e.g. blood vs plasma vs buffer).
The above situation, and how it can be corrected, is captured within Figs. 11, 12 and 13. Fig. 12 shows blood-plasma correlation without correction. In effect, for different blood or plasma doses the determined concentrations should lie on the red line - ground truth. However, due to flow related effects the determined concentration values become biased. However, by assessing the fluid flow time for each sample, a correction can be applied. This is shown in Fig. 11. Fig. 11 indicates that when the fluid flow time of a sample is below a first threshold *t_{low,RESx}* a constant correction factor equal to *C_{low,RESx}* is to be applied. Also, as indicated in Fig. 11 when the fluid flow time of a sample is above a second threshold *t_{cap,RESx}* a constant correction factor equal to *C_{high,RESx}* is to be applied. However, when the fluid flow time is between these time thresholds, then a continuously varying correction that linearly changes with time is to be applied. Fig. 13 then shows the data as shown in Fig. 12, that has been corrected on the basis of a fluid flow time as detailed in Fig. 11. This is however, a specific example of one type of correction for blood/plasma and on the basis of a fluid flow time or velocity different corrections can be applied, and for different fluids different corrections can be established and then applied.

The above description relates to a specific test developed to measure the concentration of the analyte BNP, which is a protein present in blood and plasma. However, the above description not only applies to BNP, but any type of analyte that is present in a test sample. This can be proteins, small molecules, DNA, RNA, ions, or any other type of substance that is present in a test sample.

In the above description, a blood fluid sample has been described, however the device can be used for other fluid samples such as urine, plasma, saliva, buffers, water (with contaminants), and used for assays such as for melatonin, for Jazz-materials, HNL detection. The spacing between the top part and base part of the device can be adjusted depending upon the fluid type being analysed, in order that the fluid flows between these parts under capillary action as required when a filter is not used or via wetting/capillary action when a filter is used.

In another exemplary embodiment, a computer program or computer program element is provided for controlling an appropriate system that is characterized by being configured to execute the method steps according to one of the preceding embodiments.

The computer program element might therefore be stored on a computer unit, which might also be part of an embodiment. This computing unit may be configured to perform or induce performing of the steps of the method described above. Moreover, it may be configured to operate the components of the above described apparatus. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Furthermore, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An analyser (200) for fluid sample analysis, comprising:
- at least one detecting device (210) arranged to detect physical changes; and
- a processing unit (230);
wherein, the analyser is arranged to be placed with respect to a chamber (110) configured to receive a fluid sample such that the at least one detecting device can detect physical changes in the chamber ;
wherein, the at least one detecting device is further arranged so as to measure a time for a fluid sample to move over a predetermined distance in the chamber; and
wherein, the processing unit is configured to determine at least one property of the fluid sample on the basis of the time for the fluid sample to move over the predetermined distance in the chamber.

2. Analyser according to claim 1, wherein the analyser comprises a memory (240) storing at least one relation between fluid property and at least one measurement time.

3. Analyser according to any of claims 1-2, wherein the at least one detecting device comprises at least one radiation source (40) and at least one detector (220).

4. Analyser according to claim 3, wherein the at least one radiation source and the at least one detector are configured to detect a presence of the fluid sample at an end position of the chamber to determine an end time relating to the time for the fluid sample to move over the predetermined distance in the chamber.

5. Analyser according to any of claims 3-4, wherein the at least one radiation source and the at least one detector are configured to detect a presence of the fluid sample at a start position of the chamber to determine a start time relating to the time for the fluid sample to move over the predetermined distance in the chamber.

6. Analyser according to any of claims 1-5, wherein the analyser is further configured to store at least one threshold time, and wherein the processing unit is configured to use, directly or indirectly, this at least one threshold time to determine at least one property of the fluid sample on the basis of the measured time for the fluid sample to move over the predetermined distance in the chamber.

7. Analyser according to claim 6, wherein the threshold time is determined and the processing unit is configured to determine that the fluid sample is a first type of fluid, among a plurality of types of fluids, when said measured time to move over the predetermined distance in the chamber is less than the threshold time.

8. Analyser according to any of claims 1-7, wherein the analyser comprises measurement tools (260) to measure a first concentration of an analyte in the fluid sample, and wherein the analyser is further arranged to calibrate such measurement tools by taking into account said at least one property of the fluid sample.

9. A system (300) for fluid sample analysis, comprising:
- an analyser (200) for fluid sample analysis according to any of claims 1-8; and
- a disposable (10) having a chamber (110) configured to receive a fluid sample.

10. A method (400) for fluid sample analysis, comprising:
a) receiving (410) a fluid sample in a chamber;
b) moving (420) the fluid sample in the chamber over at least a predetermined distance;
c) detecting (430) physical changes in the chamber;
d) measuring (440) a time for the fluid sample to move over the predetermined distance in the chamber; and
f) determining (460) at least one property of the fluid sample on the basis of the time for the fluid sample to move over the predetermined distance in the chamber.

11. Method according to claim 10, wherein step c) comprises transmitting radiation towards the chamber and detecting the radiation.

12. Method according to claim 11, wherein step c) comprises transmission of the radiation and the detection are arranged to detect a presence of the fluid sample at an end position of the chamber, and wherein step d) comprises using the detected presence of the fluid sample at the end position of the chamber to determine an end time relating to the time for the fluid sample to move over the predetermined distance in the chamber; and/or
wherein step c) comprises radiation transmission and detection are arranged to detect a presence of the fluid sample at a start position of the chamber, and wherein step d) comprises using the detected presence of the fluid sample at the start position of the chamber to determine a start time relating to the time for the fluid sample to move over the predetermined distance in the chamber.

13. Method according to any of claims 10-12, wherein step f) comprises using a threshold time to move over the predetermined distance in the chamber to determine at least one property of the fluid sample.

14. Method according to any of claims 10-13, wherein, the method comprises step e) determining (450) a first concentration of the fluid sample, and wherein step f) comprises determining a second concentration of the fluid sample that is the first concentration that has been adjusted on the basis of the time for the fluid sample to move over the predetermined distance in the chamber.

15. A computer program element for controlling a device according to any of claims 1 to 8 and/or system according to claim 9, which when executed by a processor is configured to carry out the method of any of claims 10-14.
